(19) Europäisches Patentamt
European Patent Office
Office européen des brevets

(11) **EP 3 722 776 B1**

(12) **EUROPÄISCHE PATENTSCHRIFT**

(45) Veröffentlichungstag und Bekanntmachung des
Hinweises auf die Patenterteilung:
**15.01.2025 Patentblatt 2025/03**

(21) Anmeldenummer: **20162905.2**

(22) Anmeldetag: **13.03.2020**

(51) Internationale Patentklassifikation (IPC):
**G01N 1/22** (2006.01)   **G01N 33/14** (2006.01)
**G01N 33/00** (2006.01)

(52) Gemeinsame Patentklassifikation (CPC):
**G01N 1/2226; G01N 33/0036; G01N 33/14;**
G01N 2001/2229

(54) **VERFAHREN ZUR MESSUNG DES SAUERSTOFFGEHALTS DES KOPFRAUMGASES IN EINER GETRÄNKEDOSE**

METHOD FOR MEASURING THE OXYGEN CONTENT OF THE HEAD SPACE GAS IN A BEVERAGE CAN

PROCÉDÉ DE MESURE DE LA TENEUR EN OXYGÈNE DU GAZ DANS L'ESPACE VIDE DANS UNE CANETTE DE BOISSONS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.04.2019 AT 503372019**

(43) Veröffentlichungstag der Anmeldung:
**14.10.2020 Patentblatt 2020/42**

(73) Patentinhaber: **Anton Paar GmbH**
**8054 Graz-Straßgang (AT)**

(72) Erfinder: **UMFER, Christof**
**8041 Graz (AT)**

(74) Vertreter: **Wildhack & Jellinek Patentanwälte GmbH**
**Landstraßer Hauptstraße 50**
**1030 Wien (AT)**

(56) Entgegenhaltungen:
**EP-A2- 0 429 396      WO-A1-2009/050530**
**US-A- 4 282 182      US-A- 4 926 681**

**Beschreibung**

Die vorliegende Erfindung betrifft ein Verfahren gemäß dem Oberbegriff des Patentanspruchs 1 sowie eine Vorrichtung zur Durchführung des Verfahrens gemäß dem Oberbegriff des Patentanspruchs 13.

Für Getränkeabfüller ist die Kenntnis des Sauerstoffgehaltes in Gebinden, wie Getränkedosen, Flaschen und dergleichen, von großem Interesse, da dieser Einfluss auf die Haltbarkeit und den Geschmack des Getränks sowie bei Metallgebinden auf deren Korrosion hat. Um Rückschlüsse auf die Ursache eines eventuell vorhandenen Sauerstoffeintrags treffen zu können, ist es wichtig, den Sauerstoffgehalt in der Flüssigkeit und im Gasraum über der Flüssigkeit, dem sogenannten Kopfraum, getrennt zu bestimmen.

Man kann auf diese Weise feststellen, ob der Sauerstoff mit der Flüssigkeit ins Gebinde gelangt ist oder durch einen eventuell schlecht justierten Füllprozess. Die Messung im Gasraum ist besonders wichtig, da aufgrund der geringen Löslichkeit von Sauerstoff in wässrigen Flüssigkeiten ein signifikanter Anteil des Sauerstoffs des Gebindes im Kopfraum bzw. dem Kopfraumgas vorliegt. So wird in der EP 0 429 396 A2 (ORBISPHERE LAB [CH]) vom 29. Mai 1991 und der WO 2009/050530 A1 (HACH ULTRA ANALYTICS SA [CH]; STEHLE GERARD [FR] ET AL.) vom 23. April 2009 ein Verfahren und ein Vorrichtung offenbart, mit dem das Kopfraumgas einer Getränkedose bzw eine Flasche durch Einbringung von Flüssigkeit in den Kopfraum entnommen und anschließend vermessen wird.

Die Messung der flüssigen Probe ist meist eine triviale Aufgabe, da die flüssige Probe über eine Schlauchleitung an einem Sauerstoffsensor vorbei aus dem Gebinde entnommen werden kann. Im Allgemeinen ist ausreichend Probe vorhanden, um solange am Sauerstoffsensor vorbeizuspülen, bis dieser nach einer Angleichzeit einen stabilen Wert anzeigt. Schwieriger ist die Situation bei der Messung des Kopfraumgases. Es sind meist nur wenige Milliliter Kopfraumgas vorhanden, wodurch es schwierig ist, dieses verwerfend am Sauerstoffsensor vorbeiströmen zu lassen.

Besonders schwierig ist die Situation bei der Messung des Kopfraumgases speziell bei Getränkedosen. Erstes Problem ist der Zugang zum Kopfraumgas. Der Dosendeckel hat vorgeprägte Strukturen, damit der Konsument die Dose öffnen kann. Häufig sind auch Logos und Schriften eingeprägt, sodass es sehr schwierig ist, am Dosendeckel gegen eine Messapparatur hin abzudichten und eine Zugangsöffnung zu stechen. Selbst wenn eine geeignete Fläche vorhanden ist, muss die Dose sehr genau ausgerichtet werden, damit diese Fläche auch zuverlässig abgedichtet wird. Der zylindrische Dosenmantel ist mechanisch zu wenig stabil um dort dichten und anstechen zu können. Damit bleibt nur noch der Boden als zuverlässig verwendbare Stelle zum Dichten und Anstechen. Der Boden bei CO2-hältigen Getränkedosen ist stets stark nach innen gewölbt, was dazu führt, dass bei am Kopf stehender Dose der tiefste Punkt des Bodens in der Flüssigkeit liegt und damit kein Zugang zum Kopfraum hergestellt werden kann. Gleichzeitig ist der tiefste Punkt des Bodens jener Punkt, an dem man am zuverlässigsten anstechen und dichten kann.

Aus dem Stand der Technik bekannte Lösungen kippen die Dose, um sie am oberen Randbereich anzustechen oder stechen durch den Deckel an, was eine genaue Justierung der Dose erforderlich macht und nicht bei allen Dosen möglich ist. Die beschriebenen bekannten Methoden erfordern höheren apparativen Aufwand und höhere Sorgfalt bzw. bessere Ausbildung des Bedienpersonals. So sind beispielsweise aus den JPH04315943 (A) und JP3405637 (B2) Vorrichtungen und Verfahren bekannt, die eine Getränkedose an deren Kante öffnen um Zugang zu dem Kopfraumgas zu erhalten.

Nachteil der aus dem Stand der Technik bekannten Verfahren ist, dass eine erhöhte Genauigkeit beim Einstellen und Anstechen der Dosen im Sampler benötigt wird. Weiters ist die Abdichtung der Anstichstelle bei den aus dem Stand der Technik bekannten Verfahren aufwendig und oft nicht sicher genug zu gewährleisten, um Messfehler durch eintretende Luft oder Sauerstoff zu verhindern.

Aufgabe der Erfindung ist es daher ein Verfahren bereitzustellen, das ermöglicht, eine Getränkedose am Boden anzustechen und dann die Kopfraummessung durchzuführen und keine erhöhten Anforderungen an die Genauigkeit beim Einstellen und Anstechen der Dosen im Sampler benötigt.

Diese Aufgabe wird durch die kennzeichnenden Merkmale des Anspruchs 1 gelöst. Dabei ist vorgesehen, dass der Flüssigkeitsspiegel in der Getränkedose über das Entnahmerohr derart abgesenkt wird, dass zwischen dem mit Kopfraumgas gefülltem Kopfraum und der Entnahmeöffnung eine direkte Verbindung besteht, wobei nach Absenken des Flüssigkeitsspiegels das im Kopfraum der Getränkedose befindliche Kopfraumgas über das Entnahmerohr und/oder den hohl ausgebildeten Piercer oder dem Anstechkopf einer Sensoreinheit, umfassend eine Anzahl von Sensoren, zugeführt wird, und derart der Sauerstoffgehalt und/oder der Sauerstoffpartialdruck und/oder das Kopfraumvolumen des Kopfraumgases durch die Sensoreinheit ermittelt wird.

Durch das Anstechen der Getränkedose am Boden ist es möglich, die Entnahmeöffnung unabhängig von einer genauen Positionierung zu stechen und dabei eine einfache Abdichtung zu erreichen. Weiters wird durch das Absenken des Flüssigkeitsspiegels der Kopfraum zugänglich gemacht oder erweitert um eine einfache Messung des Kopfraumgases zu erreichen.

Besonders vorteilhafte Ausführungsformen des erfindungsgemäßen Verfahrens werden durch die Merkmale der abhängigen Ansprüche näher definiert:
Vorteilhaft kann vorgesehen sein, dass der Flüssigkeitsspiegel in der Getränkedose durch Entnahme eines definierten Teils der in der Getränkedose befindlichen Probenflüssigkeit mittels einer Pumpe, einer Druckerhöhung mittels eines über den Piercer oder das Entnahmerohr in die Getränkedose eingebrachten Gases, insbesondere Stickstoff, oder durch den in der Getränkedose herrschenden Druck abgesenkt wird.

Der Sauerstoffgehalt der Probenflüssigkeit kann einfach ermittelt werden, indem nach der Messung des Sauerstoffgehalts des Kopfraumgases das Entnahmerohr in die in der Getränkedose befindliche Flüssigkeit abgesenkt wird und anschließend die Flüssigkeit aus der Getränkedose entnommen und in die Sensoreinheit geleitet wird und derart der in der Flüssigkeit befindliche Sauerstoffgehalt ermittelt wird.

Zur einfachen Bestimmung des Sauerstoffgehalts und/oder des Sauerstoffpartialdrucks und/oder des Kopfraumvolumens kann vorgesehen sein, dass in den durch den abgesenkten Flüssigkeitsspiegel entstanden Kopfraum, insbesondere über den hohl ausgebildeten Piercer, ein Sauerstoffsensor eingebracht wird, mit dem die Sauerstoffkonzentration bzw. der Sauerstoffpartialdruck des Kopfraumgases bestimmt wird.

In einer vorteilhaften Ausführungsform kann vorgesehen sein, dass nach Absenken des Flüssigkeitsspiegels das im Kopfraum der Getränkedose befindliche Kopfraumgas mit einer Pumpe über das Entnahmerohr und/oder den hohl ausgebildeten Piercer in die Sensoreinheit, umfassend eine Anzahl von Sensoren, und anschließend wieder zurück in den Kopfraum der Getränkedose gepumpt wird und derart der Sauerstoffgehalt und/oder der Sauerstoffpartialdruck und/oder das Kopfraumvolumen des Kopfraumgases durch die Sensoreinheit ermittelt wird. Durch diese Ausführungsform wird erreicht, dass unabhängig von dem vorhandenen Kopfraumgas der Sauerstoffgehalt in diesem einfach bestimmt werden kann und unabhängig von dem im Kopfraum freien Volumen eine Vielzahl von Sensoren verwendet werden kann. Ebenfalls steht immer genügend Kopfraumgas zur Verfügung, da dieses im Kreis bzw. wieder in den Kopfraum zurück gepumpt wird, wodurch eine genauere Bestimmung des Sauerstoffgehalts und/oder des Sauerstoffpartialdrucks und des Kopfraumvolumens erreicht wird.

Erfindungsgemäß kann vorgesehen sein, dass zur Messung des Sauerstoffgehalts, insbesondere bei der Vermessung von Getränkedosen mit schäumenden Flüssigkeiten, nach dem Absenken des Flüssigkeitsspiegels im Kopfraum der Getränkedose ein Schaum erzeugt wird, in dem das Kopfraumgas oder ein Teil des Kopfraumgases gebunden ist, wobei von dem erzeugten Schaum in der Sensoreinheit der Sauerstoffgehalt und/oder der Sauerstoffpartialdruck und/oder das Kopfraumvolumen ermittelt wird, wobei insbesondere anschließend der Schaum wieder in den Kopfraum der Getränkedose zurückgeführt wird. So kann beispielsweise, auch wenn nur ein geringer Anteil des Kopfraumgases vorhanden ist, das in dem Schaum gebundene Kopfraumgas bzw. der darin enthaltene Sauerstoff einfach ermittelt werden, da aufgrund der schlechten Löslichkeit von Sauerstoff in wässrigen Substanzen der Sauerstoffgehalt im Schaum eine sehr gute Entsprechung des Sauerstoffgehalts im Kopfraum ist.

Zur einfachen Bestimmung des Sauerstoffgehalts und/oder des Sauerstoffpartialdrucks und/oder des Kopfraumvolumens kann vorgesehen sein, dass die Sensoreinheit einen Sauerstoffsensor zur Messung des Sauerstoffgehalts und/oder des Sauerstoffpartialdrucks des Kopfraumgases aufweist, wobei insbesondere der Sauerstoffgehalt und/oder das Kopfraumvolumen durch zusätzliche Messung des Drucks mittels eines Drucksensors (und/oder Messung der Temperatur mittels eines Temperatursensors, vorzugsweise bei einer bewirkten Volumenänderung des Kopfraumgases, ermittelt wird.

Da eine Vielzahl von Sensoren temperaturempfindlich sind bzw. einen Temperaturangleich an das zu vermessende Medium erfordern, um eine höchste Genauigkeit zu erreichen, kann vorgesehen sein, dass das Kopfraumgas so oft aus dem Kopfraum in die Sensoreinheit, insbesondere an dem Temperatursensor vorbei, und anschließend wieder in den Kopfraum zurück gepumpt wird bis die Sensoreinheit, insbesondere der Drucksensor und/oder der Temperatursensor und/oder der Sauerstoffsensor, und/oder das Kopfraumgas eine stabile, vorzugsweise gleiche, Temperatur erreichen, und/oder sensorspezifische Angleichsvorgänge abgeschlossen sind. Durch das mehrmalige Umpumpen des Kopfraumgases durch die Sensoreinheit wird erreicht, dass die einzelnen Sensoren der Sensoreinheit die Temperatur des Kopfraumgases annehmen können bzw. an diese angeglichen werden und die Messungen des Kopfraumgases dann in einem angeglichenen Temperaturbereich erfolgen kann. Weiters wird derart der Temperaturangleich der Sensoren bzw. der Sensoreinheit an die Temperatur des Kopfraumgases beschleunigt. Darüber hinaus haben sonstige Angleichsprozesse wie z.B. Diffusionsprozesse im Sauerstoffsensor hinreichend Zeit für einen vollständigen Angleich.

Vorteilhaft kann vorgesehen sein, dass die Sensoreinheit eine Anzahl weiterer Sensoren, insbesondere einen $CO_2$-Sensor, einen Alkoholsensor und/oder einen Zuckersensor, aufweist, wobei mittels der weiteren Sensoren der $CO_2$ Gehalt und/oder der Alkoholholgehalt und/oder der Zuckergehalt der in der Getränkedose befindlichen Flüssigkeit bestimmt wird. Durch die Anordnung unterschiedlicher Sensoren innerhalb der Sensoreinheit bzw. innerhalb der Messanordnung können unterschiedlichste Parameter der Probenflüssigkeit bzw. des Kopfraumgases ermittelt werden, sodass eine vollständige Analyse der Probenflüssigkeit und/oder des Kopfraumgases erreicht werden kann. Alternativ kann vorgesehen sein, dass zusätzliche Sensoren außerhalb der Sensoreinheit in der Leitung bzw. der Ringleitung angeordnet sind.

Um etwaig vorhandenen Sauerstoff bzw. Verunreinigungen aus der Probenanordnung auszubringen bzw. eine Verfälschung der Messergebnisse besser verhindern zu können, kann vorgesehen sein, dass vor der Messung der Piercer, der Anstechkopf, die Sensoreinheit, die Pumpe, die Ringleitung und/oder das Entnahmerohr mit einem Spülmedium, insbesondere Stickstoff, gespült und derart von Sauerstoff und/oder Probenrückständen befreit werden.

Vorteilhaft kann vorgesehen sein, dass das Kopfraumvolumen ermittelt wird, indem der Druck in der angestochenen Getränkedose zuerst mit eingefahrenem und einmal mit ausgefahrenem Entnahmerohr gemessen wird und über die Gasgesetze und die Änderung des Drucks das Kopfraumvolumen berechnet wird.

Vorteilhaft kann vorgesehen sein, dass vor dem Anstechen des Gebindes der Druck in dem Piercer und/oder dem Anstechkopf und/oder in der Ringleitung, insbesondere durch Einbringung von Stickstoffgas, an den Innendruck des Gebindes angeglichen wird, sodass ein Aufschäumen der Probenflüssigkeit verhindert wird. So kann eine Schaumbildung einfach vermieden und ein unerwünschtes Austreten des Kopfraumgases unterbunden werden.

Es ist weiters Aufgabe der Erfindung, eine Vorrichtung zu schaffen, mit der der Sauerstoffgehalt des Kopfraumgases einfach ermittelt werden kann.

Diese Aufgabe wird durch die Merkmale des Anspruchs 13 gelöst. Dabei ist erfindungsgemäß vorgesehen, dass die Vorrichtung eine mit dem Entnahmerohr verbundene Leitung, insbesondere eine Ringleitung, aufweist, wobei innerhalb der Leitung eine Sensoreinheit angeordnet ist, mit der das Kopfraumgas einer Getränkedose über den hohl ausgebildeten Piercer entnehmbar ist, wobei die Vorrichtung derart ausgebildet ist, dass der Flüssigkeitsspiegel in der Getränkedose über das Entnahmerohr derart absenkbar ist, dass zwischen dem mit Kopfraumgas gefülltem Kopfraum und der Entnahmeöffnung eine direkte Verbindung herstellbar ist, und wobei die Vorrichtung weiters eine Steuerung umfasst, die derart ausgebildet ist, dass der Flüssigkeitsspiegel über den Piercer oder das Entnahmerohr definiert absenkbar ist bis zwischen dem mit Kopfraumgas gefülltem Kopfraum und der Entnahmeöffnung eine direkte Verbindung herstellbar ist. Mit der erfindungsgemäßen Vorrichtung kann der Sauerstoffgehalt bzw. der Sauerstoffpartialdruck und/oder das Kopfraumvolumen einfach bestimmt werden und es wird eine hohe Messgenauigkeit erreicht.

Um den Flüssigkeitsspiegel einfach absenken zu können, kann vorgesehen sein, dass die Vorrichtung Mittel zur Erhöhung des Drucks in der Getränkedose, insbesondere eine Pumpe und/oder eine Gaszufuhrvorrichtung, umfasst, wobei die Vorrichtung weiters eine Steuerung umfasst, die derart ausgebildet ist, dass der Flüssigkeitsspiegel definiert absenkbar ist.

Vorteilhaft kann vorgesehen sein, dass die Vorrichtung eine Pumpe und eine Ringleitung aufweist, wobei innerhalb der Ringleitung die Sensoreinheit angeordnet ist, mit der das Kopfraumgas der Getränkedose über den hohl ausgebildeten Piercer entnommen und über die Ringleitung, insbesondere über das Entnahmerohr, in den Kopfraum der Getränkedose zurückleitbar ist. So kann auch bei geringen Mengen von Kopfraumgas eine Angleich der Sensoren einfach erreicht werden und die Messgenauigkeit durch das Umpumpen des Kopfraumgases erhöht werden.

Vorteilhaft kann vorgesehen sein, dass die Sensoreinheit einen Sauerstoffsensor und/oder einen Drucksensor und/oder einen Temperatursensor und/oder einen $CO_2$-Sensor und/oder einen Alkoholsensor und/oder einen Zuckersensor aufweist, wobei der Sauerstoffsensor insbesondere als optochemischer Sensor nach dem Fluorescence Quenching Prinzip oder als elektrochemischer Sauerstoffsensor ausgebildet ist.

Eine effektive Umwälzung des Kopfraumgases kann einfach erreicht werden, indem die Pumpe als Umwälzpumpe, bevorzugt als Membranpumpe, Schlauchquetschpumpe, Kolbenpumpe, Zahnradpumpe Schneckenpumpe, Schaufelradpumpe oder Spritzenpumpe, ausgebildet ist.

Um ein Spülgas, wie beispielsweise Stickstoff, einfach in die Vorrichtung einbringen zu können, kann vorgesehen sein, dass die Vorrichtung eine Anzahl von in der Ringleitung integrierten Ventilen aufweist, wobei die Ventile derart in der Ringleitung angeordnet sind, dass eine automatische Reinigung der Vorrichtung, insbesondere der Ringleitung, über die Ventile erfolgen kann.

Weitere Vorteile und Ausgestaltungen der Erfindung ergeben sich aus der Beschreibung und den beiliegenden Zeichnungen.

Die Erfindung ist im Folgenden anhand von besonders vorteilhaften, aber nicht einschränkend zu verstehenden Ausführungsbeispielen in den Zeichnungen schematisch dargestellt und wird unter Bezugnahme auf die Zeichnungen beispielhaft beschrieben:

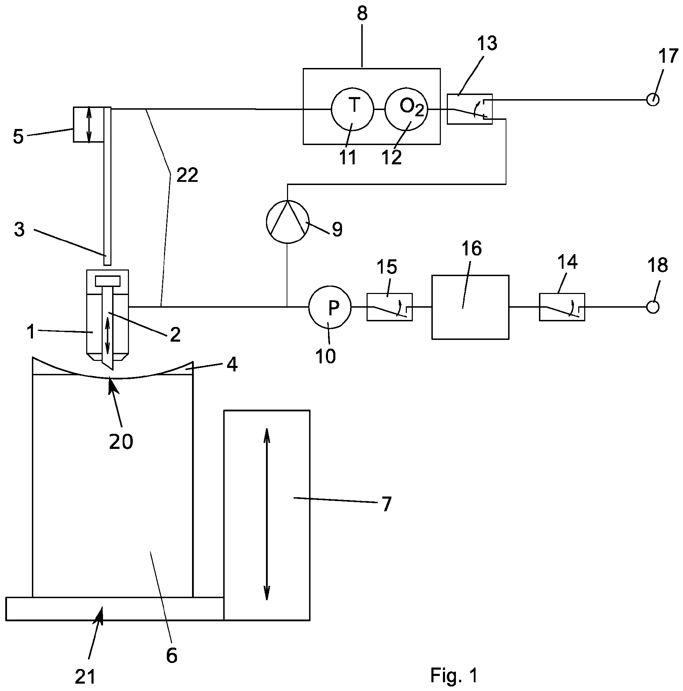

Fig. 1 zeigt eine erfindungsgemäße Vorrichtung in schematischer Darstellung.

In Fig. 1 ist eine erfindungsgemäße Vorrichtung zur Bestimmung des Sauerstoffgehalts des Kopfraumgases einer Getränkedose in schematischer Ansicht dargestellt. Die Vorrichtung umfasst einen Anstechkopf 1, auf dem ein hohl ausgebildeter Piercer 2 angeordnet ist. Der Piercer 2 ist bei dieser Ausführungsform ähnlich einer Nadel ausgebildet und kann innerhalb des Anstechkopfs 1 entlang der dargestellten Pfeile verstellt werden. Die Vorrichtung umfasst weiters eine Probenaufnahme 21, in die eine Getränkedose 6 eingebracht ist. Die Getränkedose 6 ist dabei auf dem Kopf stehend auf der Probenaufnahme 21 aufgelegt, sodass der Boden 20 der Getränkedose 6 in Richtung des Piercers 2 zeigt. Mittels eines Antriebs 7 kann die Getränkedose 6 in Richtung des Anstechkopfs 1 verstellt werden, wodurch der Anstechkopf 1 auf den Boden 20 der verschlossenen Getränkedose 6 aufgelegt bzw. angebracht werden kann. Die Vorrichtung umfasst weiters ein Entnahmerohr 3, das bei dieser Ausführungsform konzentrisch zu dem Piercer 2 angeordnet ist.

Optional kann auch eine Zentriervorrichtung oder ein Anschlag vorgesehen sein, mit dem die Getränkedose konzentrisch zum Piercer 2 angeordnet werden kann, sodass das Zentrum des Bodens der Getränkedose 6 mit dem Zentrum des Piercers 2 ausgerichtet ist.

Der Piercer 2 ist bei der bevorzugten Ausführungsform der Fig. 1 hohl ausgebildet, sodass das Entnahmerohr 3 in den Piercer 2 eindringt und durch diesen hindurch in den Kopfraum 4 gebracht werden kann. Das Entnahmerohr 3 wird dabei mittels eines Antriebes 5 verstellt. Optional kann auch vorgesehen sein, dass die Probenaufnahme 21, der Piercer 2, der Anstechkopf 1 und/oder das Entnahmerohr 3 händisch bzw. gesteuert durch einen Antrieb mit Steuerung verstellt werden können. An dem dem Anstechkopf 1 entfernten Ende des Entnahmerohrs 3 beginnt eine Ringleitung 22, die wieder in den Anstechkopf 1 zurückführt bzw. wieder in diesen mündet. In der Ringleitung 22 ist eine Sensoreinheit 8 und eine Pumpe 9 integriert. Mittels der Pumpe 9 kann eine in der Getränkedose 6 befindliche Probenflüssigkeit oder das im Kopfraum 4 der Getränkedose 6 befindliche Kopfraumgas über das Entnahmerohr 3 entnommen und so der Sensoreinheit 8 zugeführt werden. Über die Sensoreinheit 8 wird die Probenflüssigkeit oder das Kopfraumgas wieder durch die Pumpe 9 in den Anstechkopf und so wieder in die Getränkedose 6 bzw. in den Kopfraum 4 der Getränkedose 6 zurückgeführt. Optional kann auch vorgesehen sein, dass das Kopfraumgas und/oder die Probenflüssigkeit über den Anstechkopf entnommen werden und durch das Entnahmerohr 3 wieder in den Kopfraum 4 oder die Probenflüssigkeit der Getränkedose 6 zurückgeführt werden.

Optional kann an das Entnahmerohr 3, den Piercer 2 oder den Anstechkopf 1 auch eine Leitung angeordnet sein, die nicht wieder in den Kopfraum mündest, sondern in die Umgebung der Vorrichtung oder einen Auffangbehälter, in den die entnommene Probenflüssigkeit und/oder das Kopfraumgas entleert werden.

Die Sensoreinheit 8 umfasst bei der Ausführungsform der Fig. 1 einen Temperatursensor 11 sowie einen Sauerstoffsensor 12. Die Vorrichtung umfasst weiters einen Drucksensor 10, mit dem der in der Ringleitung 22 bzw. dem Kopfraum 4 der Getränkedose 6 anstehende Druck ermittelt werden kann.

Optional kann vorgesehen sein, dass die Vorrichtung bzw. die Sensoreinheit 8 auch mehrere Sauerstoffsensoren 12 umfasst, die auch außerhalb der Sensoreinheit angeordnet sein können.

Im Folgenden wird das erfindungsgemäße Verfahren anhand der Ausführungsform der Fig. 1 beispielhaft beschreiben:

Bei dem erfindungsgemäßen Verfahren wird in einem ersten Schritt die Getränkedose 6 auf der Probenaufnahme 21 platziert und dann die Probenaufnahme 21 oder der Anstechkopf 1 verstellt, sodass der Anstechkopf 1 an dem Boden 20 der Getränkedose 6 zentral ansteht. Anschließend wird der Piercer 2 in Richtung des Bodens der Getränkedose 6 verstellt, durchstößt diesen und erzeugt so eine Entnahmeöffnung in dem Boden 20 der Getränkedose 6. Mittels an dem Piercer 2 und/oder an dem Anstechkopf 1 angeordneten Dichtelementen wird die Entnahmeöffnung luftdicht abgedeckt, sodass aus der Umgebung kein Fremdgas in die Vorrichtung bzw. die Ringleitung 22 oder den Kopfraum 4 der Getränkedose 6 eindringen und kein Kopfraumgas aus der Getränkedose 6 austreten kann. Die Dichtelemente des Anstechkopfs 1 bzw. des Piercers 2 dichten dabei die Getränkedose 6 sowie die Ringleitung 22 gegen die Umgebung der Vorrichtung luftdicht ab. Das Entnahmerohr 3 wird anschließend in die Dose 6 abgesenkt. Da bei Getränkedosen 6 durch die Krümmung des Bodens 20 meist keine Verbindung zwischen dem Kopfraum und der Entnahmeöffnung besteht, wird das Entnahmerohr 3 zuerst in die Flüssigkeit eingebracht und ein Teil der Flüssigkeit solange aus der Getränkedose 6 entnommen, bis eine direkte Verbindung zwischen der Entnahmeöffnung und dem Kopfraum 4 der Getränkedose besteht, sodass das Kopfraumgas über das Entnahmerohr und der Ringleitung 22 der Sensoreinheit 8 bzw. dem Sauerstoffsensor 12, dem Drucksensor 10 und dem Temperatursensor 11 zugeführt werden kann. Die Entnahme der Probenflüssigkeit kann dabei über ein Ventil 13 und der Öffnung 17 in die Umgebung oder einen Auffangbehälter erfolgen. Nun ist der Kopfraum 4 frei zugänglich und kann einer Sauerstoffmessung zugeführt werden. Durch die künstliche Vergrößerung des Kopfraums 4 bleibt die Sauerstoffmasse im Kopfraum 4 unverändert, wodurch das Messergebnis durch das Absenken des Flüssigkeitsspiegels nicht verfälscht wird. Nach dem Absenken des Flüssigkeitsspiegels kann das vergrößerte Kopfraumvolumen ermittelt werden und dieses zur Berechnung der Sauerstoffmasse bzw des Sauerstoffgehalts im Kopfraumgas aus dem gemessenen Sauerstoffpartialdruck herangezogen werden. Die Menge der zu entnehmenden Flüssigkeit ist abhängig von der Getränkedose 6 und der nominalen Füllhöhe und kann z.B. empirisch ermittelt werden, indem man mit dem beschriebenen Verfahren für eine bestimmte Anzahl an Sekunden Probe entnimmt und anschließend den Füllstand prüft.

In einer optionalen Ausführungsform wird das Kopfraumvolumen vor der Flüssigkeitsentnahme gemessen und aus diesem und dem Durchmesser der Getränkedose 6 berechnet, wie viel Flüssigkeit entnommen werden muss, um den Zugang zwischen Kopfraum 4 und Entnahmeöffnung zu ermitteln. Die Entnahme kann bei bekannter Durchflussrate durch das Entnahmerohr 3 oder die Leitung bzw. Ringleitung 22 zeitgesteuert oder über eine beliebige Form einer Durchflussmessung erfolgen.

In der apparativ einfachsten Form kann das Kopfraumvolumen optional ermittelt werden, indem der Druck in der angestochenen Getränkedose einmal mit eingefahrenem und einmal mit ausgefahrenem Entnahmerohr 3 gemessen wird. Durch das Zusatzvolumen des Entnahmerohrs 3 ändert sich der Druck des Kopfraumgases bzw der Druck in der Getränkedose und die Gasgesetze erlauben bei bekanntem Volumen des Piercers 2 bzw. des Entnahmerohrs 3 eine Berechnung des Kopfraumvolumens.

Als Ideal hat sich ein Restfüllstand nach der Absenkung des Flüssigkeitsspiegels von ca. 5 mm tiefer als der tiefste Punkt des Bodens 20 der Getränkedose 6 erwiesen.

EP 3 722 776 B1

Über den Antrieb 5 wird nach Absenken des Flüssigkeitsspiegels das Entnahmerohr 3 so positioniert, dass dieses in den vergrößerten Kopfraum 4 hineinragt, ohne in die Flüssigkeit einzutauchen. Mittels der Pumpe 9 wird das Kopfraumgas aus dem Kopfraum 4 abgepumpt und über die Ringleitung 22 der Sensoreinheit 8 zugeführt. In der Sensoreinheit 8 wird mittels des Temperatursensors 11 und des Sauerstoffsensors 12 der Sauerstoffgehalt sowie die Temperatur des Kopfraumgases bestimmt. Mittels des Drucksensors 10 wird weiters der im Kopfraum 4 anstehende Druck ermittelt und dann beispielsweise über die Gasgleichungen das Volumen des Kopfraumgases bestimmt. Über die Ringleitung 22 wird dann durch die Pumpe 9 das Kopfraumgas von der Sensoreinheit 8 wieder über den Anstechkopf 1 in den Kopfraum 4 der Getränkedose 6 zurückbefördert. Durch das Umpumpen des Kopfraumgases über die Ringleitung 22 wird so ein Kreislauf des Kopfraumgases innerhalb der Vorrichtung bewirkt, sodass das Kopfraumgas einfach oder auch mehrfach an der Sensoreinheit 8 bzw. den Sensoren der Vorrichtung vorbei gepumpt werden kann. Durch das einfache oder mehrfache Vorbeipumpen des Kopfraumgases an der Sensoreinheit 8 bzw. den Sensoren wird ein Temperaturangleich der Sensoren verbessert und/oder die Abhängigkeit des Messergebnisses vom meist asymptotischen Angleichsverhalten der Sensoren, insbesondere des Sauerstoffsensors 12, reduziert, sodass die Bestimmung des Sauerstoffgehalts, des Sauerstoffpartialdrucks und/oder des Kopfraumvolumens beschleunigt und der Messwert genauer bestimmt werden kann. Geeignete Sauerstoffsensoren aus dem Stand der Technik messen beispielsweise den Sauerstoffgehalt nach Diffusion des Sauerstoffs durch eine Membran, dies kann Angleichszeiten bei der Diffusion von einigen Sekunden erforderlich machen, auch können sich passivierende Sauerstoffschichten vor dem jeweiligen Sensor bilden, die eine Fehlmessung begünstigen. Durch das einfache oder mehrfache Vorbeipumpen des Kopfraumgases an der Sensoreinheit 8 bzw. den Sensoren wird die Diffusion verbessert und ebenfalls vermieden, dass passivierende Sauerstoffschichten gebildet werden.

Nach der Bestimmung des Sauerstoffgehalts des Kopfraumgases bzw. des Kopfraumvolumens oder des Sauerstoffpartialdrucks kann das Entnahmerohr 3 aus dem Kopfraum 4 weiter in die Probenflüssigkeit der Getränkedose abgesenkt werden. Anschließend wird die Probenflüssigkeit zur Sensoreinheit 8 gepumpt und auch der Sauerstoffgehalt, die Temperatur bzw. der Druck der Probenflüssigkeit bestimmt.

Alternativ kann vorgesehen sein, dass die Vorrichtung anstelle der Ringleitung 22 eine Leitung aufweist, die in einen Auffangbehälter oder ähnlichem mündet und die Probenflüssigkeit beim messen der Sauerstoffkonzentration in der Flüssigkeit an der Sensoreinheit 8 vorbei und/oder beim Absenken des Flüssigkeitsspiegels verwerfend entnommen wird.

Der Flüssigkeitsspiegel in der Getränkedose 6 kann mittels einer Pumpe 9 oder durch eine Druckerhöhung mittels eines über den Piercer 2 oder das Entnahmerohr 3 oder dem Anstechkopf 1 in die Getränkedose 6 eingebrachten Gases abgesenkt werden. Ein geeignetes Gas ist dabei jegliches Gas, das die Sauerstoffkonzentration in der Getränkedose 6 nicht verändert, wie z.B Stickstoff. Alternativ kann der Flüssigkeitsspiegel, durch den in der Getränkedose herrschenden Druck abgesenkt wird, dies ist insbesondere bei Getränkedosen 6 mit kohlesäurehaltigen Getränken vorgesehen.

Problematisch ist, dass Getränkedosen 6 mit schäumenden Flüssigkeiten, zum Beispiel Bier, dazu neigen, nach dem Anstechen und dem Absenken des Flüssigkeitsspiegels mittels des Piercers 2, insbesondere bei schnellem Einlegen in den Probenhalter 21, zu schäumen. In dem entstandenen Schaum ist ein Teil des Kopfraumgases örtlich nahezu stationär gebunden, sodass dieser gebundene Gasanteil nicht an dem Messprozess über die Ringleitung 22 beteiligt ist. Optional kann daher bei schäumenden Flüssigkeiten bewusst Schaum erzeugt bzw. die Schaumbildung gefördert werden, sodass das gesamte Kopfraumgas homogen im Schaum gebunden wird. Die Schaumbildung kann beispielsweise erfolgen, indem das Entnahmerohr 3 über den Antrieb 5 wenige Millimeter unterhalb der Flüssigkeitsoberfläche positioniert wird und das Kopfraumgas vom Anstechkopf 2 über die Pumpe 9 und das Entnahmerohr 3 in die Flüssigkeitsoberfläche gepumpt wird und dort Schaum erzeugt. Anschließend kann der erzeugte Schaum mittels der Pumpe 9 über die Ringleitungen 22 der Sensoreinheit 8 zugeführt werden und so der Sauerstoffgehalt des Schaums ermittelt werden. Da der Sauerstoffgehalt des Schaums aufgrund der schlechten Löslichkeit von Sauerstoff in der Probenflüssigkeit dem des Kopfraumes 4 bzw. des Kopfraumgases entspricht, kann so der Sauerstoffgehalt des Kopfraumgases bestimmt werden.

Die richtige Einstellung des Gasdrucks im Piercer 2 beim Anstechen des Bodens 20 der Getränkedose 6 ist je nach Getränkedose und darin befindlicher Probenflüssigkeit unterschiedlich. Vor allem bei Proben, die - meist bei niedrigen Temperaturen - zur Ausbildung eines steifen Schaums neigen, muss der Druck des im Piercer 2 anliegenden Gases auf die Flüssigkeit abgestimmt werden. Wird nämlich der Piercerdruck bzw. der Druck des Piercers 2 deutlich höher als der Innendruck der Getränkedose 6 gewählt, dann schießt beim Anstechen Stickstoff durch die Flüssigkeit in den Kopfraum und bildet dabei Schaum, der den Sauerstoff im Kopfraum immobilisiert und damit der späteren Messung entzieht. Der Druck im Piercer 2 ist daher bevorzugt niedriger als der Doseninnendruck. Erst wenn soviel Flüssigkeit entnommen wurde, dass der Kopfraum durch die gestochene Öffnung zugänglich ist, kann der Druck über die Ventile 14, 15, und das Speichervolumen 16 bzw. die Pumpe 9 erhöht werden und so der Flüssigkeitsspiegel weiter abgesenkt werden bzw. das Kopfraumgas entnommen werden.

Da die Sensoren meist ein asymtotisches Angleichsverhalten mit einer sensorspezifischen Angleichszeit sowie ein temperaturabhängiges Messverhalten haben, ist es vorteilhaft, dass die Sensoren, insbesondere der Sauerstoffsensor 12 und der Temperatursensor 11, an die Temperatur bzw. Konzentration des Kopfraumgases bzw. der Probenflüssigkeit angeglichen werden. Um diesen Angleich rasch durchführen zu können, wird optional das Kopfraumgas 4 mehrfach an der Sensoreinheit 8 bzw. an den Sensoren vorbei gepumpt und so der Angleich beschleunigt. Durch die Umwälzung des Kopfraumgases bzw. durch das mehrfache Umpumpen des Kopfraumgases können so auch geringe Mengen des Kopfraumgases mittels der Sensoreinheit 8 vermessen werden bzw. auch bei geringen Mengen des Kopfraumgases ein rascher Angleich der Sensoren an das Kopfraumgas und die Probenflüssigkeit erreicht werden. Dieser Angleich kann alle zeitabhängigen Effekte wie den Temperaturangleich und/oder die Diffusion des Messgases durch die Membran des Sensors etc. umfassen.

Die Vorrichtung umfasst weiters ein in der Ringleitung 22 angeordnetes Ventil 13, das mit einer Leitung, die in die Umgebung an einer Öffnung 17 führt, verbunden ist. Über die Öffnung 17 kann beispielsweise ein Spülgas wie Stickstoff oder eine Reinigungslösung in die Ringleitung bzw. zu der Sensoreinheit 8 bzw. der Pumpe 9 und den Sensoren gelangen und so Probenrückstände oder Restsauerstoff aus der Vorrichtung ausgespült werden.

Optional kann vorgesehen sein, dass, wie in Fig. 1 dargestellt, die Vorrichtung eine Anzahl von weiteren Ventilen 14, 15 sowie einen Speichervolumen 16 umfasst. Das Speichervolumen 16 ist über das Ventil 15 mit dem Drucksensor 10 verbunden und über ein weiteres Ventil 14 mit der Umgebung der Vorrichtung verbunden. Da das ursprünglich nur im Kopfraum 4 befindliche Kopfraumgas beim Öffnen der Getränkedose 6 oder nach dem Absenken des Flüssigkeitsspiegels durch den Piercer 2 sich in der Ringleitung 22 verteilen kann, werden niedrigere $O_2$ Konzentrationen gemessen als im Kopfraum 4 des ursprünglich geschlossenen Getränkedose 6 vorhanden war. Dieser systematische Fehler wird rechnerisch korrigiert. Dazu ist die Kenntnis des Pumpkreisvolumens bzw. des Volumens der Ringleitungen 22 und der daran angeschlossenen Komponenten notwendig und die Kenntnis des Kopfraumvolumens. Das Kopfraumvolumen wird im Zuge des Messablaufs durch die Sensoreinheit 8 bzw. den Drucksensor 10 und den Temperatursensor 11 messtechnisch bestimmt und/oder über die Anwendung der Gasgesetze ermittelt.

Dazu ist in der Vorrichtung der Ausführungsform der Fig. 1 ein leeres Speichervolumen 16 integriert. In einem ersten Schritt vor dem Anstechen der Getränkedose 6 wird das Ventil 14, das über einen Lufteingang 18 mit der Umgebung der Vorrichtung verbunden ist, geöffnet und das Speichervolumen 16 auf Umgebungs-Luftdruck gebracht. Wenn auch das Ventil 15, das das Speichervolumen mit der Ringleitung 22 verbindet, geöffnet ist, kann ein erster Luftdruck p2 gemessen werden. Nun werden die Ventile 14 und 15 geschlossen. Nach dem Anstechen der Getränkedose 6 durch den Piercer 2 wird der Druck p1 gemessen, der sich aus der Kombination des Drucks der angestochenen Getränkedose 6 und dem im Anstechkopf 1 und den Ringleitungen 22 anliegenden Druck ergibt. Dann wird das Ventil 15 geöffnet und der sich dann einstellende Mischdruck p3 gemessen. Bei bekanntem Speichervolumen 16 kann nun das Kopfraumvolumen $V_{Headspace}$ unter Anwendung des Boyle Mariotte'schen Gesetzes (Gleichung 1) berechnet werden.

$$V_{Headspace} = V_{Expansion} \times \frac{p_3 - p_2}{p_1 - p_3} - V_{Apparat} \quad \text{Gleichung 1}$$

Da die Expansion weder rein isotherm noch rein adiabatisch abläuft, stellt das Ergebnis nur eine gute Näherung dar.

Expansionsvolumen $V_{Expansion}$ und Apparatvolumen $V_{Apparat}$, also das in der Vorrichtung bzw. in der Ringleitung 22, dem Entnahmerohr 3, dem Piercer 2 und dem Anstechkopf 1 befindliche Volumen, können aus der Konstruktion bestimmt werden, besser ist allerdings, wenn mit verschiedenen bekannten Kopfraumvolumina $V_{Headspace}$ eine Messreihe durchgeführt und daraus $V_{Expansion}$ und $V_{Apparat}$ berechnet werden. Die beiden Werte enthalten dann neben den bekannten konstruktiven Geometrieinformationen auch Korrekturen für Abweichungen vom isothermen Verhalten und können so ein noch genaueres Ergebnis der Messung ermöglichen.

Die gemessene Sauerstoffkonzentration kann dann mit den bekannten Volumina anhand der Gleichung 2 korrigiert werden.

$$O_{2,corr} = O_{2,gemessen} \times \frac{V_{Apparat} + V_{Headspace}}{V_{Headspace}} \quad \text{Gleichung 2}$$

Alternativ kann man am Beginn der Messung das Speichervolumen 16 auch auf einen höheren Druck als den, der in der Getränkedose 6 herrscht, bringen. Dazu wird der Anstechkopf 1 vor dem Anstechen mit dem Piercer 2 zur Getränkedose 6 abgedichtet. Dann werden die Ventile 14 und 15 geöffnet, sodass im gesamten Bereich zwischen dem Lufteingang 18 und Anstechkopf 1 derselbe Druck herrscht. Dieser Druck wird mit dem Drucksensor 10 gemessen. Anschließend werden die Ventile 14 und 15 geschlossen und der Druck damit im Speichervolumen 16 "eingesperrt". Das restliche Verfahren zur Messung der Sauerstoffkonzentration wird dann analog zu dem oben beschriebenen Verfahren durchgeführt.

Alternativ kann vorgesehen sein, dass das Kopfraumgas bzw. die Probenflüssigkeit über den Piercer 2 in die Ringleitung 22 abgepumpt wird oder das Entnahmerohr 3 direkt an den Piercer 2 anschließt. Alternativ kann der Piercer 2 nach dem Öffnen bzw. Durchstechen des Bodens 20 der Getränkedose 6 in dem Kopfraum 4 verbleiben und über diesen das Kopfraumgas bzw. die Probenflüssigkeit in die Ringleitung 22 gepumpt werden bzw. der Flüssigkeitsspiegel über den Piercer 2 abgesenkt werden.

Optional kann die Sensoreinheit 8 oder die Vorrichtung auch eine Anzahl weiterer Sensoren beispielsweise einen $CO_2$-Sensor, einen Alkoholsensor, einen Zuckersensor und/oder weitere Sensoren aufweisen, die in der Ringleitung 22 oder der Sensoreinheit 8 integriert sind. Mittels der weiteren Sensoren kann beispielsweise der $CO_2$-Gehalt oder der Alkoholgehalt oder der Zuckergehalt der Probenflüssigkeit bestimmt werden und so weitere Parameter der Probenflüssigkeit ermittelt werden. Die weiteren Sensoren können optional auch über die Öffnung 17 mit der Probenflüssigkeit befüllt werden. Die weiteren Sensoren können beispielsweise bei der Produktion von Getränken wie Bier oder Limonaden weitere Informationen liefern, sodass mittels der erfindungsgemäßen Vorrichtung die Qualitätskontrolle des Abfüllprozesses oder des Produktionsprozesses einfach überwacht werden kann.

Der Sauerstoffsensor 12 kann insbesondere als optochemischer Sensor nach dem Fluorescence Quenching Prinzip oder beispielsweise als elektrochemischer Sauerstoffsensor ausgebildet sein. Optional zu der in Fig. 1 dargestellten Ausführungsform kann die Sensoreinheit auch nur einen Sauerstoffsensor 12 umfassen, mit dem der Sauerstoffgehalt des Kopfraumgases und/oder der Probenflüssigkeit bestimmt wird.

Die Pumpe 9, der in Fig. 1 dargestellten Ausführungsform kann beispielsweise als Umwälzpumpe, insbesondere als Membranpumpe, Schlauchquetschpumpe, Kolbenpumpe, Zahnradpumpe, Schneckenpumpe, Schaufelradpumpe oder Spritzenpumpe ausgebildet sein.

Optional können der Antrieb 7 bzw. die Verstellmechanismen des Piercers 2 und des Entnahmerohrs 3 händisch oder auch anders angetrieben werden und so die Verstellung der einzelnen Teile zueinander bewirkt werden.

## Patentansprüche

1. Verfahren zur Messung des Sauerstoffgehalts des Kopfraumgases in einer Getränkedose (6), mit insbesondere gekrümmten Boden (20),

   - wobei die Getränkedose (6) auf dem Kopf stehend, mit dem Boden (20) entgegen der Schwerkraft angeordnet wird, sodass sich das Kopfraumgas im Bereich des Bodens sammelt,
   - wobei mit einem auf einem Anstechkopf (1) angeordneten hohl ausgebildeten Piercer (2) eine Entnahmeöffnung in den Boden (20), insbesondere in das Zentrum des Bodens (20), der Getränkedose (6) eingebracht wird, in die ein Entnahmerohr (3) eindringt und wobei die Entnahmeöffnung mittels am Piercer (2) oder dem Anstechkopf (1) angeordneten Dichtelementen luftdicht abgedeckt wird,
   **dadurch gekennzeichnet, dass** der Flüssigkeitsspiegel in der Getränkedose (6) über das Entnahmerohr (3) derart abgesenkt wird, dass zwischen dem mit Kopfraumgas gefülltem Kopfraum (4) und der Entnahmeöffnung eine direkte Verbindung besteht,
   wobei nach Absenken des Flüssigkeitsspiegels das im Kopfraum (4) der Getränkedose (6) befindliche Kopfraumgas über das Entnahmerohr (3) und/oder den hohl ausgebildeten Piercer (2) oder dem Anstechkopf (1) einer Sensoreinheit (8), umfassend eine Anzahl von Sensoren, zugeführt wird, und derart der Sauerstoffgehalt und/oder der Sauerstoffpartialdruck und/oder das Kopfraumvolumen des Kopfraumgases durch die Sensoreinheit (8) ermittelt wird.

2. Verfahren nach Anspruch 1, **dadurch gekennzeichnet, dass** der Flüssigkeitsspiegel in der Getränkedose (6) durch Entnahme eines definierten Teils der in der Getränkedose (6) befindlichen Probenflüssigkeit mittels einer Pumpe (9), einer Druckerhöhung mittels eines über den Piercer (2) oder das Entnahmerohr (3) in die Getränkedose (6) eingebrachten Gases, insbesondere Stickstoff, oder durch den in der Getränkedose herrschenden Druck abgesenkt wird.

3. Verfahren nach Anspruch 1 oder 2, **dadurch gekennzeichnet, dass** nach der Messung des Sauerstoffgehalts des Kopfraumgases das Entnahmerohr (3) in die in der Getränkedose (6) befindliche Flüssigkeit abgesenkt wird und anschließend die Flüssigkeit aus der Getränkedose (6) entnommen und in die Sensoreinheit (8) geleitet wird und derart der in der Flüssigkeit befindliche Sauerstoffgehalt ermittelt wird.

4. Verfahren nach einem der vorangehenden Ansprüche, **dadurch gekennzeichnet, dass** in den durch den abgesenkten Flüssigkeitsspiegel entstanden Kopfraum (4), insbesondere über den hohl ausgebildeten Piercer (2), ein Sauerstoffsensor (12) eingebracht wird, mit dem der die Sauerstoffkonzentration bzw. der Sauerstoffpartialdruck des Kopfraumgases bestimmt wird.

**5.** Verfahren nach einem der vorangehenden Ansprüche **dadurch gekennzeichnet, dass** nach Absenken des Flüssigkeitsspiegels das im Kopfraum (4) der Getränkedose (6) befindliche Kopfraumgas mit einer Pumpe (9) über das Entnahmerohr (3) und/oder den hohl ausgebildeten Piercer (2) in die Sensoreinheit (8), umfassend eine Anzahl von Sensoren, und anschließend wieder zurück in den Kopfraum (4) der Getränkedose (6) gepumpt wird und derart der Sauerstoffgehalt und/oder der Sauerstoffpartialdruck und/oder das Kopfraumvolumen des Kopfraumgases durch die Sensoreinheit (8) ermittelt wird.

**6.** Verfahren nach einem der vorangehenden Ansprüche, **dadurch gekennzeichnet, dass** zur Messung des Sauerstoffgehalts, insbesondere bei der Vermessung von Getränkedosen (6) mit schäumenden Flüssigkeiten, nach dem Absenken des Flüssigkeitsspiegels im Kopfraum (4) der Getränkedose (6) ein Schaum erzeugt wird, in dem das Kopfraumgas oder ein Teil des Kopfraumgases gebunden ist, wobei von dem erzeugten Schaum in der Sensoreinheit (8) der Sauerstoffgehalt und/oder der Sauerstoffpartialdruck und/oder das Kopfraumvolumen ermittelt wird, wobei insbesondere anschließend der Schaum wieder in den Kopfraum (4) der Getränkedose (6) zurückgeführt wird.

**7.** Verfahren nach einem der vorangehenden Ansprüche, **dadurch gekennzeichnet, dass** die Sensoreinheit (8) einen Sauerstoffsensor (12) zur Messung des Sauerstoffgehalts und/oder des Sauerstoffpartialdrucks des Kopfraumgases aufweist, wobei insbesondere der Sauerstoffgehalt und/oder das Kopfraumvolumen durch zusätzliche Messung des Drucks mittels eines Drucksensors (10) und/oder Messung der Temperatur mittels eines Temperatursensors (11), vorzugsweise bei einer bewirkten Volumenänderung des Kopfraumgases, ermittelt wird.

**8.** Verfahren nach einem der Ansprüche 5 bis 7, **dadurch gekennzeichnet, dass** das Kopfraumgas so oft aus dem Kopfraum (4) in die Sensoreinheit (8), insbesondere an dem Temperatursensor (11) vorbei, und anschließend wieder in den Kopfraum zurück gepumpt wird bis die Sensoreinheit (8), insbesondere der Drucksensor (10) und/oder der Temperatursensor (11) und/oder der Sauerstoffsensor (12), und/oder das Kopfraumgas eine stabile, vorzugsweise gleiche, Temperatur erreichen, und/oder sensorspezifische Angleichsvorgänge abgeschlossen sind.

**9.** Verfahren nach einem der vorangehenden Ansprüche, **dadurch gekennzeichnet, dass** die Sensoreinheit (8) eine Anzahl weiterer Sensoren, insbesondere einen $CO_2$-Sensor, einen Alkoholsensor und/oder einen Zuckersensor, aufweist, wobei mittels der weiteren Sensoren der $CO_2$ Gehalt und/oder der Alkoholholgehalt und/oder der Zuckergehalt der in der Getränkedose befindlichen Flüssigkeit bestimmt wird.

**10.** Verfahren nach einem der vorangehenden Ansprüche, **dadurch gekennzeichnet, dass** vor der Messung der Piercer (2), der Anstechkopf (1), die Sensoreinheit (8), die Pumpe (9), die Ringleitung (22) und/oder das Entnahmerohr (3) mit einem Spülmedium, insbesondere Stickstoff, gespült und derart von Sauerstoff und/oder Probenrückständen befreit werden.

**11.** Verfahren nach einem der vorangehenden Ansprüche, **dadurch gekennzeichnet, dass** das Kopfraumvolumen ermittelt wird, indem der Druck in der angestochenen Getränkedose (6) zuerst mit eingefahrenem und einmal mit ausgefahrenem Entnahmerohr (3) gemessen wird und über die Gasgesetze und die Änderung des Drucks das Kopfraumvolumen berechnet wird.

**12.** Verfahren nach einem der vorangehenden Ansprüche, **dadurch gekennzeichnet, dass** vor dem Anstechen des Gebindes (6) der Druck in dem Piercer (3) und/oder dem Anstechkopf (1) und/oder in der Ringleitung (22), insbesondere durch Einbringung von Stickstoffgas, an den Innendruck des Gebindes (6) angeglichen wird, sodass ein Aufschäumen der Probenflüssigkeit verhindert wird.

**13.** Vorrichtung zur Bestimmung des Sauerstoffgehalts des Kopfraumgases in einer flüssigkeitsgefüllten Getränkedose (6), insbesondere mit einem Verfahren nach einem der Ansprüche 1 bis 9, wobei die Vorrichtung einen Anstechkopf (1) aufweist, an dem ein, insbesondere hohl ausgebildeter, Piercer (2) und ein Entnahmerohr (3) angeordnet sind, wobei die Vorrichtung eine mit dem Entnahmerohr (3) verbundene Leitung, insbesondere eine Ringleitung (22), aufweist, wobei innerhalb der Leitung eine Sensoreinheit (8) angeordnet ist, mit der das Kopfraumgas einer Getränkedose (6) über den hohl ausgebildeten Piercer (2) entnehmbar ist, **dadurch gekennzeichnet, dass** die Vorrichtung derart ausgebildet ist, dass der Flüssigkeitsspiegel in der Getränkedose (6) über das Entnahmerohr (3) derart absenkbar ist, dass zwischen dem mit Kopfraumgas gefülltem Kopfraum (4) und der Entnahmeöffnung eine direkte Verbindung herstellbar ist, und wobei die Vorrichtung weiters eine Steuerung umfasst, die derart ausgebildet ist, dass der Flüssigkeitsspiegel über den Piercer (2) definiert absenkbar ist bis eine zwischen dem mit Kopfraumgas gefülltem Kopfraum (4) und der Entnahmeöffnung eine direkte Verbindung herstellbar ist.

**14.** Vorrichtung nach Anspruch 13, **dadurch gekennzeichnet, dass** die Vorrichtung Mittel zur Erhöhung des Drucks in der Getränkedose (6), insbesondere eine Pumpe (9) und/oder eine Gaszufuhrvorrichtung, umfasst, wobei die Vorrichtung weiters eine Steuerung umfasst, die derart ausgebildet ist, dass der Flüssigkeitsspiegel definiert absenkbar ist,

und/oder

dass die Vorrichtung eine Pumpe (9) und eine Ringleitung (22) aufweist, wobei innerhalb der Ringleitung (22) die Sensoreinheit (8) angeordnet ist, mit der das Kopfraumgas der Getränkedose (6) über den hohl ausgebildeten Piercer (2) entnommen und über die Ringleitung (22), insbesondere über das Entnahmerohr (3), in den Kopfraum (4) der Getränkedose (6) zurückleitbar ist.

**15.** Vorrichtung nach Anspruch 13 bis 14, dadurch gegenzeichnet, dass die Sensoreinheit (8) einen, Sauerstoffsensor (12) und/oder einen Drucksensor (10) und/oder einen Temperatursensor (11) und/oder einen CO2-Sensor und/oder einen Alkoholsensor und/oder einen Zuckersensor aufweist, wobei der Sauerstoffsensor (12) insbesondere als optochemischer Sensor nach dem Fluorescence Quenching Prinzip oder als elektrochemischer Sauerstoffsensor ausgebildet ist,

und/oder

**dadurch gekennzeichnet, dass** die Pumpe (9) als Umwälzpumpe, bevorzugt als Membranpumpe, Schlauchquetschpumpe, Kolbenpumpe, Zahnradpumpe Schneckenpumpe, Schaufelradpumpe oder Spritzenpumpe, ausgebildet ist,

und/oder

**dadurch gekennzeichnet, dass** die Vorrichtung eine Anzahl von in der Ringleitung (22) integrierten Ventilen (13, 14, 15) aufweist, wobei die Ventile (13, 14, 15) derart in der Ringleitung (22) angeordnet sind, dass eine automatische Reinigung der Vorrichtung, insbesondere der Ringleitung, über die Ventile (13, 14, 15) erfolgen kann.

**Claims**

**1.** Method for measuring the oxygen content of the headspace gas in a beverage can (6), with in particular a curved bottom (20),

- wherein the beverage can (6) is arranged upside down with the bottom (20) arranged counter to the force of gravity, such that the headspace gas collects in the region of the bottom,
- wherein a hollow piercer (2) arranged on a piercing head (1) is used to form a sampling opening in the bottom (20), in particular in the centre of the bottom (20) of the beverage can (6), into which a sampling tube (3) penetrates and wherein the sampling opening is covered in an airtight manner by means of sealing elements arranged on the piercer (2) or the piercing head (1),

**characterized in that** the liquid level in the beverage can (6) is lowered by way of the sampling tube (3) in such a way that a direct connection is established between the headspace (4) that is filled with headspace gas and the sampling opening,

wherein, after lowering the liquid level, the headspace gas contained in the headspace (4) of the beverage can (6) is supplied to a sensor unit (8), comprising a number of sensors, via the sampling tube (3) and/or the hollow piercer (2) or the piercing head (1), and the oxygen content and/or the oxygen partial pressure and/or the headspace volume of the headspace gas is thus determined by the sensor unit (8).

**2.** Method according to claim 1, **characterized in that** the liquid level in the beverage can (6) is lowered by sampling a defined portion of the sample liquid contained in the beverage can (6) by means of a pump (9), by increasing the pressure by means of introducing a gas, in particular nitrogen, into the beverage can (6) via the piercer (2) or the sampling tube (3), or by the pressure prevailing in the beverage can.

**3.** Method according to claim 1 or 2, **characterized in that**, after the measurement of the oxygen content of the headspace gas, the sampling tube (3) is lowered into the liquid contained in the beverage can (6) and then the liquid is sampled from the beverage can (6) and passed into the sensor unit (8) and the oxygen content contained in the liquid is thus determined.

**4.** Method according to any one of the preceding claims, **characterized in that** an oxygen sensor (12) is introduced into the headspace (4) created by the lowered liquid level, in particular via the hollow piercer (2), through which the oxygen concentration or the oxygen partial pressure of the headspace gas is determined.

5. Method according to any one of the preceding claims, **characterized in that**, after the liquid level has been lowered, the headspace gas contained in the headspace (4) of the beverage can (6) is pumped by a pump (9) via the sampling tube (3) and/or the hollow piercer (2) into the sensor unit (8), comprising a number of sensors, and then back into the headspace (4) of the beverage can (6) and the oxygen content and/or the oxygen partial pressure and/or the headspace volume of the headspace gas is thus determined by the sensor unit (8).

6. Method according to any one of the preceding claims, **characterized in that** in order to measure the oxygen content, in particular when measuring beverage cans (6) with foaming liquids, a foam is generated in the headspace (4) of the beverage can (6) after the liquid level has been lowered, in which foam the headspace gas or a portion of the headspace gas is bound, wherein the oxygen content and/or the oxygen partial pressure and/or the headspace volume is determined from the generated foam in the sensor unit (8), wherein, in particular, the foam is then returned to the headspace (4) of the beverage can (6).

7. Method according to any one of the preceding claims, **characterized in that** the sensor unit (8) has an oxygen sensor (12) for measuring the oxygen content and/or the oxygen partial pressure of the headspace gas, wherein in particular the oxygen content and/or the headspace volume is determined by additional measurement of the pressure by means of a pressure sensor (10) and/or measurement of the temperature by means of a temperature sensor (11), preferably when a change in volume of the headspace gas is brought about.

8. Method according to any one of claims 5 to 7, **characterized in that** the headspace gas is pumped from the headspace (4) into the sensor unit (8), in particular past the temperature sensor (11), and then back into the headspace until the sensor unit (8), in particular the pressure sensor (10) and/or the temperature sensor (11) and/or the oxygen sensor (12), and/or the headspace gas reach a stable, preferably equal, temperature, and/or sensor-specific adjustment processes are completed.

9. Method according to any one of the preceding claims, **characterized in that** the sensor unit (8) has a number of further sensors, in particular a $CO_2$ sensor, an alcohol sensor and/or a sugar sensor, wherein the $CO_2$ content and/or the alcohol content and/or the sugar content of the liquid in the beverage can is determined by means of the further sensors.

10. Method according to any one of the preceding claims, **characterized in that**, before the measurement, the piercer (2), the piercing head (1), the sensor unit (8), the pump (9), the ring line (22) and/or the sampling tube (3) are flushed with a flushing medium, in particular nitrogen, and thus freed from oxygen and/or sample residues.

11. Method according to any one of the preceding claims, **characterized in that** the headspace volume is determined by measuring the pressure in the pierced beverage can (6) first with the sampling tube (3) inserted and once with the sampling tube (3) withdrawn, and by calculating the headspace volume using the gas laws and the change in pressure.

12. Method according to any one of the preceding claims, **characterized in that** before the container (6) is pierced, the pressure in the piercer (3) and/or the piercing head (1) and/or in the ring line (22) is adjusted to the internal pressure of the container (6), in particular by introducing nitrogen gas, such that foaming of the sample liquid is prevented.

13. Device for determining the oxygen content of the headspace gas in a liquid-filled beverage can (6), in particular by a method according to any one of claims 1 to 9, wherein the device has a piercing head (1) on which a piercer (2), in particular a hollow piercer, and a sampling tube (3) are arranged,

wherein the device has a line connected to the sampling tube (3), in particular a ring line (22), wherein within the line a sensor unit (8) is arranged, with which the headspace gas of a beverage can (6) can be sampled via the hollow piercer (2),
**characterized in that** the device is configured in such a way that the liquid level in the beverage can (6) can be lowered via the sampling tube (3) such that a direct connection between the headspace (4) that is filled with headspace gas and the sampling opening can be established, and wherein the device further comprises a controller which is configured such that the liquid level can be lowered in a defined manner via the piercer (2) until a direct connection can be established between the headspace (4) that is filled with headspace gas and the sampling opening.

14. Device according to claim 13, **characterized in that** the device comprises means for increasing the pressure in the beverage can (6), in particular a pump (9) and/or a gas supply device, wherein the device further comprises a controller which is configured such that the liquid level can be lowered in a defined manner,
and/or
the device has a pump (9) and a ring line (22), wherein within the ring line the sensor unit (8) is arranged, with which the headspace gas of the beverage can (6) can be sampled via the hollow piercer (2) and can be returned to the headspace (4) of the beverage can (6) via the ring line (22), in particular via the sampling tube (3).

15. Device according to claim 13 to 14, **characterized in that** the sensor unit (8) has an oxygen sensor (12) and/or a pressure sensor (10) and/or a temperature sensor (11) and/or a C02 sensor and/or an alcohol sensor and/or a sugar sensor, wherein the oxygen sensor (12) is configured in particular as an optochemical sensor according to the fluorescence quenching principle or as an electrochemical oxygen sensor,
and/or

   **characterized in that** the pump (9) is configured as a circulation pump, preferably as a diaphragm pump, peristaltic pump, piston pump, gear pump, worm pump, paddle wheel pump or syringe pump, and/or
   **characterized in that** the device has a number of valves (13, 14, 15) integrated in the ring line (22), wherein the valves (13, 14, 15) are arranged in the ring line (22) in such a way that the device, in particular the ring line, can be cleaned automatically via the valves (13, 14, 15).


**Revendications**

1. Procédé de mesure du taux d'oxygène du gaz d'espace vide dans une canette de boisson (6), avec en particulier un fond incurvé (20),

   - dans lequel la canette de boisson (6) est disposée à l'envers, avec le fond (20) contre la force de gravité, de sorte que le gaz de l'espace vide s'accumule au niveau du fond,
   - dans lequel une ouverture de prélèvement est pratiquée dans le fond (20), en particulier au centre du fond (20) de la canette de boisson (6) au moyen d'un perceur creux (2) disposé sur une tête de perçage (1), canette dans laquelle un tube de prélèvement (3) pénètre, et dans lequel l'ouverture de prélèvement est recouverte hermétiquement au moyen d'éléments d'étanchéité disposés sur le perceur (2) ou la tête de perçage (1),

      **caractérisé en ce que** le niveau de liquide dans la canette de boisson (6) est abaissé par l'intermédiaire du tube de prélèvement (3) de sorte qu'il existe une connexion directe entre l'espace vide (4) rempli de gaz d'espace vide et l'ouverture de prélèvement,
      dans lequel, après abaissement du niveau de liquide, le gaz d'espace vide se trouvant dans l'espace vide (4) de la canette de boisson (6) est amené, par l'intermédiaire du tube de prélèvement (3) et/ou du perceur creux (2) ou de la tête de perçage (1), à une unité de capteur (8) comprenant un certain nombre de capteurs, et le taux d'oxygène et/ou la pression partielle d'oxygène et/ou le volume d'espace vide du gaz d'espace vide est ainsi déterminé par l'unité de capteur (8).

2. Procédé selon la revendication 1, **caractérisé en ce que** le niveau de liquide dans la canette de boisson (6) est abaissé par prélèvement d'une partie définie du liquide d'échantillon se trouvant dans la canette de boisson (6) au moyen d'une pompe (9), par augmentation de pression au moyen d'un gaz, en particulier de l'azote, introduit dans la canette de boisson (6) par le perceur (2) ou le tube de prélèvement (3), ou par pression régnant dans la canette de boisson.

3. Procédé selon la revendication 1 ou 2, **caractérisé en ce qu'**après la mesure du taux d'oxygène du gaz d'espace vide, le tube de prélèvement (3) est abaissé dans le liquide se trouvant dans la canette de boisson (6), puis le liquide est prélevé de la canette de boisson (6) et est guidé dans l'unité de capteur (8) et le taux d'oxygène se trouvant dans le liquide est ainsi déterminé.

4. Procédé selon l'une quelconque des revendications précédentes, **caractérisé en ce qu'**un capteur d'oxygène (12) est introduit dans l'espace vide (4) créé par le niveau de liquide abaissé, en particulier par l'intermédiaire du perceur creux (2), avec lequel la concentration en oxygène ou la pression partielle d'oxygène du gaz d'espace vide est déterminée.

5. Procédé selon l'une quelconque des revendications précédentes, **caractérisé en ce qu'**après abaissement du niveau de liquide, le gaz d'espace vide se trouvant dans l'espace vide (4) de la canette de boisson (6) est pompé avec une pompe (9) dans l'unité de capteur (8), comprenant un certain nombre de capteurs, par l'intermédiaire du tube de prélèvement (3) et/ou du perceur creux (2), puis renvoyé dans l'espace vide (4) de la canette de boisson (6), et le taux d'oxygène et/ou la pression partielle d'oxygène et/ou le volume d'espace vide du gaz d'espace vide est ainsi déterminé par l'unité de capteur (8).

6. Procédé selon l'une quelconque des revendications précédentes, **caractérisé en ce que** pour mesurer le taux d'oxygène, en particulier lors de la mesure de canettes de boisson (6) avec des liquides moussants, une mousse est générée après l'abaissement du niveau de liquide dans l'espace vide (4) de la canette de boisson (6), mousse dans laquelle le gaz d'espace vide ou une partie du gaz d'espace vide est lié, dans lequel le taux d'oxygène et/ou la pression partielle d'oxygène et/ou le volume d'espace vide est déterminé à partir de la mousse générée dans l'unité de capteur (8), dans lequel la mousse est en particulier ensuite réintroduite dans l'espace vide (4) de la canette de boisson (6).

7. Procédé selon l'une quelconque des revendications précédentes, **caractérisé en ce que** l'unité de capteur (8) présente un capteur d'oxygène (12) pour mesurer le taux d'oxygène et/ou la pression partielle d'oxygène du gaz d'espace vide, dans lequel en particulier le taux d'oxygène et/ou le volume d'espace vide est déterminé par mesure supplémentaire de la pression au moyen d'un capteur de pression (10) et/ou une mesure de la température au moyen d'un capteur de température (11), de préférence lors d'une modification de volume provoquée du gaz d'espace vide.

8. Procédé selon l'une quelconque des revendications 5 à 7, **caractérisé en ce que** le gaz d'espace vide est pompé de l'espace vide (4) dans l'unité de capteur (8), en particulier en passant devant le capteur de température (11), puis est renvoyé dans l'espace vide jusqu'à ce que l'unité de capteur (8), en particulier le capteur de pression (10) et/ou le capteur de température (11) et/ou le capteur d'oxygène (12), et/ou le gaz d'espace vide atteignent une température stable, de préférence identique, et/ou que les processus d'ajustement spécifiques aux capteurs soient terminés.

9. Procédé selon l'une quelconque des revendications précédentes, **caractérisé en ce que** l'unité de capteur (8) présente un certain nombre de capteurs supplémentaires, en particulier un capteur de $CO_2$, un capteur d'alcool et/ou un capteur de sucre, dans lequel le taux de $CO_2$ et/ou le taux d'alcool et/ou le taux de sucre du liquide se trouvant dans la canette de boisson est déterminé au moyen des capteurs supplémentaires.

10. Procédé selon l'une quelconque des revendications précédentes, **caractérisé en ce que**, avant la mesure, le perceur (2), la tête de perçage (1), l'unité de capteur (8), la pompe (9), la ligne annulaire (22) et/ou le tube de prélèvement (3) sont rincés avec un agent de rinçage, en particulier de l'azote, et sont ainsi débarrassés d'oxygène et/ou de résidus d'échantillon.

11. Procédé selon l'une quelconque des revendications précédentes, **caractérisé en ce que** le volume d'espace vide est déterminé en mesurant la pression dans la canette de boisson percée (6), d'abord avec le tube de prélèvement (3) inséré et une fois avec le tube de prélèvement (3) retiré, et en calculant le volume d'espace vide à l'aide des lois des gaz et de la variation de la pression.

12. Procédé selon l'une quelconque des revendications précédentes, **caractérisé en ce qu'**avant de percer le conteneur (6), la pression dans le perceur (3) et/ou la tête de perçage (1) et/ou la ligne annulaire (22) est ajustée à la pression interne du conteneur (6), en particulier par l'introduction d'azote gazeux, de sorte qu'un moussage du liquide d'échantillon est évité.

13. Dispositif pour déterminer le taux d'oxygène du gaz d'espace vide dans une canette de boisson (6) remplie de liquide, en particulier avec un procédé selon l'une quelconque des revendications 1 à 9, dans lequel le dispositif présente une tête de perçage (1) sur laquelle un perceur (2), en particulier creux, et un tube de prélèvement (3) sont disposés,

dans lequel le dispositif présente une conduite, en particulier une ligne annulaire (22), reliée au tube de prélèvement (3), dans lequel une unité de capteur (8) est disposée à l'intérieur de la conduite, unité de capteur avec laquelle le gaz d'espace vide d'une canette de boisson (6) peut être prélevé par l'intermédiaire du perceur creux (2),
**caractérisé en ce que** le dispositif est conçu de telle manière que le niveau de liquide dans la canette de boisson (6) puisse être abaissé par l'intermédiaire du tube de prélèvement (3) de sorte qu'une connexion directe puisse être établie entre l'espace vide (4) rempli de gaz d'espace vide et l'ouverture de prélèvement, et dans lequel le dispositif comprend en outre un dispositif de commande qui est conçu de sorte que le niveau de liquide puisse être abaissé de manière définie via le perceur (2) jusqu'à ce qu'une connexion directe puisse être établie entre l'espace vide (4) rempli de gaz d'espace vide et l'ouverture de prélèvement.

14. Dispositif selon la revendication 13, **caractérisé en ce que** le dispositif comprend des moyens pour augmenter la pression dans la canette de boisson (6), en particulier une pompe (9) et/ou un dispositif d'alimentation en gaz, dans lequel le dispositif comprend en outre un dispositif de commande qui est conçu de manière à ce que le niveau de liquide puisse être abaissé de manière définie,

et/ou

**en ce que** le dispositif présente une pompe (9) et une ligne annulaire (22), dans lequel l'unité de capteur (8) est disposée à l'intérieur de la ligne annulaire (22), unité de capteur avec laquelle le gaz d'espace vide de la canette de boisson (6) peut être prélevé via le perceur creux (2) et renvoyé dans l'espace vide (4) de la canette de boisson (6) par l'intermédiaire de la ligne annulaire (22), en particulier par l'intermédiaire du tube de prélèvement (3).

15. Dispositif selon les revendications 13 à 14, **caractérisé en ce que** l'unité de capteur (8) présente un capteur d'oxygène (12) et/ou un capteur de pression (10) et/ou un capteur de température (11) et/ou un capteur de CO2 et/ou un capteur d'alcool et/ou un capteur de sucre, dans lequel le capteur d'oxygène (12) est en particulier un capteur optochimique selon le principe du quenching ou un capteur d'oxygène électrochimique,

et/ou

**caractérisé en ce que** la pompe (9) est conçue en tant que pompe de circulation, de préférence en tant que pompe à membrane, pompe péristaltique, pompe à piston, pompe à engrenages, pompe à vis, pompe à palettes ou pompe à seringue, et/ou

**caractérisé en ce que** le dispositif présente un certain nombre de soupapes (13, 14, 15) intégrées dans la ligne annulaire (22), dans lequel les soupapes (13, 14, 15) sont disposées dans la ligne annulaire (22) de sorte qu'un nettoyage automatique du dispositif, en particulier de la ligne annulaire, puisse être effectué par l'intermédiaire des soupapes (13, 14, 15).

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0429396 A2
- WO 2009050530 A1, HACH ULTRA ANALYTICS SA [CH]; STEHLE GERARD [FR]
- JP H04315943 A
- JP 3405637 B